# EUROPÄISCHE PATENTSCHRIFT

(11) **EP 0 687 464 B1**
(45) Veröffentlichungstag und Bekanntmachung des Hinweises auf die Patenterteilung: **05.09.2001**
(21) Anmeldenummer: 95109188.3
(22) Anmeldetag: 14.06.1995
(51) Int. Cl.: A61K 9/46, A61K 47/10

(54) **Verfahren zur Herstellung von Brausetabletten**
Process for manufacturing effervescent tablets
Procédé de fabrication de comprimés effervescents

(30) Priorität: 15.06.1994 DE 4420735
(43) Veröffentlichungstag der Anmeldung: 20.12.1995
(73) Patentinhaber: Machoczek, Horst, 37130 Gleichen/Rheinhausen (DE)
(72) Erfinder: Machoczek, Horst, 37130 Gleichen/Rheinhausen (DE)
(74) Vertreter: Grüter, Klaus, Dipl.-Phys.

(56) Entgegenhaltungen:
- EP-A- 0 070 127
- EP-A- 0 219 337
- EP-A- 0 395 329
- FR-A- 2 409 789
- US-A- 4 725 427
- PATENT ABSTRACTS OF JAPAN vol. 11, no. 141 (C-421), 8.Mai 1987 & JP-A-61 281183 (KAO CORP.), 11.Dezember 1986,
- DATABASE WPI Week 7133 Derwent Publications Ltd., London, GB; AN 71-54222s XP002010971 & JP-B-46 028 094 (TOKYO FINE CHEMICAL CO. LTD.)
- DATABASE WPI Week 8326 Derwent Publications Ltd., London, GB; AN 83-62638k XP002010972 & JP-A-58 085 816 (CHIYODA YAKUHIN KK) , 23.Mai 1983

## Beschreibung

Die Erfindung bezieht sich auf ein Verfahren zur Herstellung von Brausetabletten nach dem Oberbegriff des Patentanspruches 1.

Bei der Herstellung von Brausetabletten ist es bekannt, Granulate als Vorstufe für die Brausetabletten auszubilden. Eine solche Granulierung ist jedoch mit Schwierigkeiten verbunden, da die Brausezusätze einer Brausetablette extrem feuchteempfindlich sind, und beispielsweise eine Zugabe von Wasser zu den Brausezusätzen immer mit einer Freisetzung von in den Brausezusätzen gebundenem Kohlendioxid einhergeht.

Aus der US-A-4 725 427 ist ein Verfahren bekannt, bei dem als Wirkstoffe wasserlösliche Vitamine in Pulverform miteinander vermischt, gesiebt und mit pulverisierter Lactose vermischt werden. Fettlösliche Vitamine werden in einer Alkohollösung bestehend aus 60 bis 80 % Ethylalkohol und 20 bis 40 % Propylenglykol aufgelöst und mit der Mischung aus wasserlöslichen Vitaminen und pulverisierter Lactose vermischt. In einem anschließenden Trocknungsvorgang wird der Wirkstoffmischung der Ethylalkohol entzogen. Nach einem erneuten Siebvorgang wird die getrocknete Mischung mit Brausezusätzen - Zitronensäure und Bicarbonat - vermischt und durch Pressen tablettiert.

Nachteilig bei dem bekannten Verfahren ist, dass ein relativ aufwendiger Trocknungsvorgang notwendig ist, um den zuvor zugesetzten Ethylalkohol zu entfernen. Mit dem Trocknungsprozess werden zudem auch Anteile des Propylenglykols wieder entfernt, so dass relativ große Mengen Propylenglykol benötigt werden. Der Trocknungsprozess kann zudem teilweise zur Zersetzung von bestimmten Phytopharmaka führen.

Neben den auf einer Granulierung der Einsatzstoffe aufbauenden Verfahren sind auch solche Verfahren zur Herstellung von Brausetabletten bekannt, bei denen die Einsatzstoffe direkt tablettiert werden. Ein solches Verfahren wird beispielsweise in der EP-A-0 219 337 beschrieben. Dabei wird nach dem Gegenstand dieser älteren Patentanmeldung pulverförmige Dextrose oder Sucrose als Bindemittel für die Brausetabletten verwendet. In Vergleichsbeispielen wird dieses Bindemittel mit verschiedenen Zuckeraustauschstoffen als Bindemittel vergleichen. Bei der Verwendung von Dextrose und / oder Sucrose als Bindemittel beträgt der Bindemittelanteil 10 bis 40 % des Gesamtgewichts der Brausetabletten.

Der Erfindung liegt die Aufgabe zugrunde, ein Verfahren zur Herstellung von stabilen, sich mit hoher Auflösegeschwindigkeit auszeichnenden Brausetabletten aufzuzeigen, dass mit geringem Aufwand durchführbar ist, bei dem beliebige Wirkstoffe oder Wirkstoffkombinationen verarbeitbar sind und bei dem nur ein geringer Bindemittelanteil am Gesamtgewicht der Brausetabletten erforderlich ist.

Diese Aufgabe wird erfindungsgemäß in Verbindung mit dem Oberbegriff des Anspruches 1 dadurch gelöst, dass als Bindemittel Propylenglykol oder Glycerin ohne Zusatz von Lösungsmitteln mit einem Bindemittelanteil von 0,04 bis 2,5 % des Gesamtgewichtes der Brausetablette verwendet wird.

Propylenglykol oder Glycerin als Bindemittel sind in sehr kleinen Anteilen ausreichend, um bei den Brausetabletten die gewünschten mechanischen Eigenschaften bei Einhaltung des gewünschten Auflösungsverhaltens zu erreichen. Auch die Handhabung des Bindemittels Propylenglykol bzw. Glycerin ist sehr einfach. Es ist mit dem Wirkstoff oder der Wirkstoffkombination und ggf. den Trägerstoffen mischbar, ohne das deren Schüttfähigkeit und damit deren leichte Mischfähigkeit mit den Brausezusätzen verloren geht. Gleichzeitig besteht keine Gefahr, dass das Bindemittel zu einem Kohlendioxidverlust bei den Brausezusätzen führt.

Die bei dem erfindungsgemäßen Verfahren einsetzbaren Wirkstoffe sind in keiner Weise eingeschränkt. Sie schließen beispielsweise Calcium-, Magnesium-, Kalium-, Eisen-II-Salze, Vitamin E, Vitamin C, Paracetamol, Cimetidin, Piracetam, Acetylsalicylsäure, Ambroxol, Indometacin und Acetylcystein sowie beliebige andere Wirkstoffe ein.

Die verwendbaren Wirkstoffkombinationen umfassen u. a. Multivitamine, Mulivitamine mit Mineralsalzen, Betacarotin mit Vitamin E und / oder Vitamin C, Vitamin C mit Mineralsalzen, anionisch und / oder nicht-ionische Tenside und andere
(weiter auf Seite 3 der ursprünglichen Anmeldungsunterlagen) waschaktive Substanzen. Daneben sind sämtlich oral in Lösungsform verabreichbare Wirkstoffkombinationen einsetzbar.

Die ggfs. einzusetzenden Trägerstoffe können Geschmackskorrigenzien, beispielsweise Süßstoffe, Zuckeraustauschstoffe, Aromen, oder zusätzlich oder alternativ einzusetzende weitere Trägerstoffe, wie Farb- oder Duftstoffe, sein. Entsprechende Trägerstoffe sind dem Fachmann in großer Zahl bekannt. Ihre Auswahl ist abhängig vom gewünschten Ergebnis, insbesondere hinsichtlich des Geschmacks der aufgelösten Brausetabletten und liegt im Bereich des Wissens des Fachmanns.

Dem Fachmann sind geeignete Zusammensetzungen der Brausezusätze für die Brausetabletten ebenfalls bekannt. Derartige Brausezusätze bestehen aus einer Basenkomponente und einer Säurekomponente, wobei insbesondere die Säurekomponente gleichzeitig als Wirkstoff Verwendung finden kann. Bekanntes Beispiel hierfür ist Ascorbinsäure (Vitamin C). Üblicherweise verwendete und auch hier geeignete Basenkomponenten sind Natriumcarbonat, Natriumhydrogencarbonat, Kaliumcarbonat, Kaliumhydrogencarbonat und Calciumcarbonat. Als Säurekomponenten kommen neben Ascorbinsäure Mononatriumcitrat, Weinsäure und/oder Citronensäure in Betracht.

Vorteilhafte Ausgestaltungen des erfindungsgemäßen Verfahrens sind in den Unteransprüchen 2 bis 8 beschrieben.

Ein besonderer Vorteil des Verfahrens zur Herstellung der Brausetabletten ist der geringe apparative und auch zeitliche Aufwand bei gleichzeitiger großer Freiheit hinsichtlich der eingesetzten Wirkstoffe bzw. Wirkstoffkombinationen. So ist das Verfahren sowohl geeignet zur Herstellung pharmazeutischer Brausetabletten als auch von Diät-Brausetabletten als auch von Brausetabletten in Form von Waschmittel-, Bade- und Entkalkungstabletten.

Die Erfindung wird durch die nachfolgenden Ausführungsbeispiele näher erläutert.

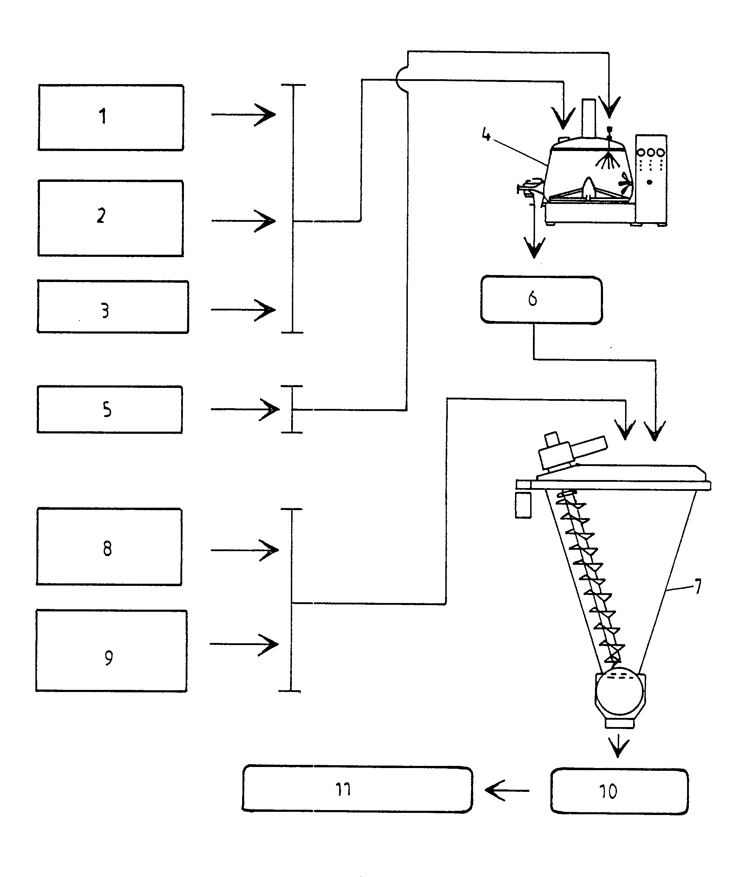

Dabei entspricht das Vorgehen in allen Beispielen dem in der Figur schematisch wiedergegebenen Verfahrensablauf. Der Wirkstoff bzw. die Wirkstoffkombination 1 wird zusammen mit Süßstoffen, Füllstoffen und Farbstoffen 2 sowie Aromastoffen 3 in einem Intensivmischer 4 vermischt. Anschließend wird das Bindemittel 5 aufgesprüht und nochmals intensiv vermischt. Dann wird der Inhalt des Intensivmischers 4 über ein Frewitt 6 mit einer Siebweite von 1 mm in einen Chargenmischer 7 überführt. In dem Chargenmischer 7 werden die Brausezusätze in Form einer Säurekomponente 8 und einer Basenkomponente 9 zugegeben. Nach dem Mischen im Chargenmischer 7 wird die Tablettiermischung der Tablettierung 10 zugeführt. Die fertigen Brausetabletten gelangen anschließend zur Röhrchenabfüllung 11.

### Beispiel 1.1

Acetylsalicylsäure 500 mg Brausetablette

| | |
|---|---|
| 100,000 kg | Acetylsalicylsäure |
| 10,000 kg | primäres Natriumphosphat |
| 4,000 kg | Tylose C 300 P |

werden im Intensivmischer 3 min gemischt.

0,100 kg Propylenglycol werden zugegeben und nochmals kurzzeitig gemischt.

Das Acetylsalicylsäure-Konzentrat wird im klimatisierten Raum (relative Luftfeuchtigkeit < 25 %) in einem geeigneten Mischer mit

| | |
|---|---|
| 186,000 kg | Citronensäure |
| 240,000 kg | Natriumhydrogencarbonat |

ca. 10 bis 15 min gemischt.

Die resultierenden 539,900 kg Tablettiermischung werden auf einer Tablettenpresse unter externer Schmierung mit weniger als 0,100 kg Magnesiumstearat zu 200.000 Rundtabletten mit 22 mm Durchmesser und einem Tablettengewicht von 2,7 g verpreßt.

Die Tablettenhärte beträgt 45 bis 55 N.

### Beispiel 1.2

### Acetylsalicylsäure + Vitamin C Brausetablette

| | |
|---|---|
| 75,000 kg | Acetylsalicylsäure |
| 18,750 kg | Ascorbinsäure |
| 3,000 kg | primäres Natriumphosphat |
| 1,350 kg | Natriumsacharin |
| 4,500 kg | Fruchtaroma |

werden im Intensivmischer 3 min gemischt.

0,075 kg Propylenglycol werden zugegeben und nochmals kurzzeitig gemischt.

Das Acetylsalicylsäure-Vitamin C-Süßstoff-Aromakonzentrat wird im klimatisierten Raum (relative Luftfeuchtigkeit < 25 %) in einem geeigneten Mischer mit

| | |
|---|---|
| 182,400 kg | Citronensäure |
| 254,850 kg | Natriumbicarbonat |

ca. 10 bis 15 min gemischt.

Die resultierenden 539,925 kg Tablettiermischung werden auf einer Tablettenpresse unter externer Schmierung mit 0,075 kg Magnesiumstearat zu 150.000 Rundtabletten mit 25 mm Durchmesser und einem Tablettengewicht von 3,6 g verpreßt.

Die Tablettenhärte beträgt nach dem Tablettieren >50 N und nach weiteren 24 h >100 N.

### Beispiel 2.1

### Paracetamol 500 mg Brausetablette

| | |
|---|---|
| 25,000 kg | Paracetamol |
| 1,750 kg | Natriumsaccharinat |
| 15,000 kg | Maltodextrin |
| 4,000 kg | Fruchtaroma |

werden im Intensivmischer 3 min gemischt.

0,150 kg Propylenglycol werden zugegeben und nochmals kurzzeitig gemischt.

Das Paracetamol-Süßstoff-Aromakonzentrat wird im klimatisierten Raum (relative Luftfeuchtigkeit < 25 %) in einem geeigneten Mischer mit

| | |
|---|---|
| 35,000 kg | Citronensäure |
| 69,000 kg | Natriumbicarbonat |

ca. 10 bis 15 min gemischt.

Die resultierenden 149,900 kg Tablettiermischung werden auf einer Tablettenpresse unter externer Schmierung mit weniger als 0,100 kg Magnesiumstearat zu 50.000 Rundtabletten mit 22 mm Durchmesser und einem Tablettengewicht von 3 g verpreßt.

### Beispiel 2.2

### Paracetamol + Vitamin C Brausetablette

| | |
|---|---|
| 25,000 kg | Paracetamol |
| 10,000 kg | Ascorbinsäure |
| 1,750 kg | Natriumsacharinat |
| 7,500 kg | Maltodextrin |
| 7,500 kg | Karion Instant |
| 4,000 kg | Fruchtaroma |

werden im Intensivmischer 3 min gemischt.

0,150 kg Propylenglycol werden zugegeben und nochmals kurzzeitig gemischt.

Das Paracetamol-Vitamin C-Süßstoff-Aromakonzentrat wird im klimatisierten Raum (relative Luftfeuchtigkeit < 25 %) in einem geeigneten Mischer mit

| | |
|---|---|
| 41,000 kg | Citronensäure |
| 53,250 kg | Natriumbicarbonat |

ca. 10 bis 15 min gemischt.

Die resultierenden 150,150 kg Tablettiermischung werden auf einer Tablettenpresse unter externer Schmierung mit 0,100 kg Magnesiumstearat zu 50.000 Rundtabletten mit 22 mm Durchmesser und einem Tablettengewicht von 3 g verpreßt.

Die Tablettenhärte beträgt 30 bis 50 N.

### Beispiel 3.1

### Acetylcystein 100 mg Brausetablette

| | |
|---|---|
| 5,000 kg | Acetylcystein |
| 1,000 kg | Aspartam |
| 2,000 kg | Fruchtaroma |

werden im Intensivmischer 3 min gemischt.

0,0075 kg Propylenglycol werden zugegeben und nochmals kurzzeitig gemischt.

Das Acetylcystein-Süßstoff-Aromakonzentrat wird im klimatisierten Raum (relative Luftfeuchtigkeit < 25 %) in einem geeigneten Mischer mit

| | |
|---|---|
| 78,950 kg | Citronensäure |
| 58,030 kg | Natriumbicarbonat |

ca. 10 bis 15 min gemischt.

Die resultierenden 144,975 kg Tablettiermischung werden auf einer Tablettenpresse unter externer Schmierung mit weniger als 0,025 kg Magnesiumstearat zu 50.000 Rundtabletten mit 22 mm Durchmesser und einem Tablettengewicht von 2,9 g verpreßt.

Die Tablettenhärte beträgt 40 bis 50 N.

### Beispiel 3.2

### Acetylcystein 400 mg Brausetablette

| | |
|---|---|
| 20,000 kg | Acetylcystein |
| 1,000 kg | Aspartam |
| 2,500 kg | Fruchtaroma |

werden im Intensivmischer 3 min gemischt.

0,006 kg Propylenglycol werden zugegeben und nochmals kurzzeitig gemischt.

Das Acetylcystein-Süßstoff-Aromakonzentrat wird im klimatisierten Raum (relative Luftfeuchtigkeit < 25 %) in einem geeigneten Mischer mit

| | |
|---|---|
| 66,650 kg | Citronensäure |
| 52,841 kg | Natriumbicarbonat |

ca. 10 bis 15 min gemischt.

Die resultierenden 142,977 kg Tablettiermischung werden auf einer Tablettenpresse unter externer Schmierung mit weniger als 0,025 kg Magnesiumstearat :zu 50.000 Rundtabletten mit 22 mm Durchmesser und einem Tablettengewicht von 2,86 g verpreßt.

Die Tablettenhärte beträgt 40 bis 60 N.

### Beispiel 4.1

### Ambroxol 30 mg Brausetablette

| | |
|---|---|
| 3,000 kg | Ambroxol |
| 0,400 kg | Natriumsaccharinat |
| 1,125 kg | Aspartam |
| 0,030 kg | Kollidon 25 |
| 0,050 kg | Macrogol 6000 |
| 25,510 kg | Maltodextrin |
| 25,510 kg | Karion Instant |
| 5,000 kg | Fruchtaroma |

werden im Intensivmischer 3 min gemischt.

0,375 kg Propylenglycol werden zugegeben und nochmals kurzzeitig gemischt.

Das Ambroxol-Farbstoff-Süßstoff-Aromakonzentrat wird im klimatisierten Raum (relative Luftfeuchtigkeit < 25 %) in einem geeigneten Mischer mit

| | |
|---|---|
| 118,850 kg | Citronensäure |
| 100,150 kg | Natriumbicarbonat |

ca. 10 bis 15 min gemischt.

Die resultierenden 280,000 kg Tablettiermischung werden auf einer Tablettenpresse unter externer Schmierung mit weniger als 0,050 kg Magnesiumstearat zu 100.000 Rundtabletten mit 22 mm Durchmesser und einem Tablettengewicht von 2,8 g verpreßt.

Die Tablettenhärte sofort nach dem Tablettieren beträgt >50 N.

### Beispiel 4.2

### Ambroxol 60 mg Brausetablette

| | |
|---|---|
| 6,000 kg | Ambroxol |
| 0,500 kg | Natriumsaccharinat |
| 1,100 kg | Aspartam |
| 0,030 kg | Kollidon 25 |
| 0,050 kg | Macrogol 6000 |
| 23,160 kg | Maltodextrin |
| 23,160 kg | Karion Instant |
| 6,000 kg | Fruchtaroma |

werden im Intensivmischer 3 min gemischt.

0,400 kg Propylenglycol werden zugegeben und nochmals kurzzeitig gemischt.

Das Ambroxol-Farbstoff-Süßstoff-Aromakonzentrat wird im klimatisierten Raum (relative Luftfeuchtigkeit < 25 %) in einem geeigneten Mischer mit

| | |
|---|---|
| 119,180 kg | Citronensäure |
| 100,420 kg | Natriumbicarbonat |

ca. 10 bis 15 min gemischt.

Die resultierenden 280,000 kg Tablettiermischung werden auf einer Tablettenpresse unter externer Schmierung mit weniger als 0,050 kg Magnesiumstearat zu 100.000 Rundtabletten mit 22 mm Durchmesser und einem Tablettengewicht von 2,8 g verpreßt.

Die Tablettenhärte sofort nach dem Tablettieren beträgt >50 N.

### Beispiel 5

### Cimetidin 200 mg Brausetablette

| | |
|---|---|
| 10,000 kg | Cimetidin |
| 2,000 kg | Natriumcyclamat |
| 0,500 kg | Natriumsaccharinat |
| 20,000 kg | Sorbitol |
| 8,000 kg | Fruchtaroma |

werden im Intensivmischer 3 min gemischt.

0,500 kg Propylenglycol werden zugegeben und nochmals kurzzeitig gemischt.

Das Ambroxol-Farbstoff-Süßstoff-Aromakonzentrat wird im klimatisierten Raum (relative Luftfeuchtigkeit < 25 %) in einem geeigneten Mischer mit

| | |
|---|---|
| 42,500 kg | Citronensäure |
| 75,500 kg | Natriumbicarbonat |

ca. 10 bis 15 min gemischt.

Die resultierenden 159,000 kg Tablettiermischung werden auf einer Tablettenpresse unter externer Schmierung mit weniger als 0,075 kg Magnesiumstearat zu 100.000 Rundtabletten mit 22 mm Durchmesser und einem Tablettengewicht von 3,18 g verpreßt.

Die Tablettenhärte beträgt 40 bis 70 N.

### Beispiel 6.1

### Vitamin C 225 mg Brausetablette

| | |
|---|---|
| 11,250 kg | Ascorbinsäure |
| 1,000 kg | Natriumcyclamat |
| 0,350 kg | Natriumsaccharinat |
| 0,005 kg | Riboflavin |
| 20,000 kg | Sorbitol |
| 2,500 kg | Maltodextrin |
| 0,500 kg | Fruchtaroma |

werden im Intensivmischer 3 min gemischt.

0,125 kg Propylenglycol werden zugegeben und nochmals kurzzeitig gemischt.

Das Ascorbinsäure-Farbstoff-Süßstoff-Aromakonzentrat wird im klimatisierten Raum (relative Luftfeuchtigkeit < 25 %) in einem geeigneten Mischer mit

| | |
|---|---|
| 94,145 kg | Citronensäure |
| 70,000 kg | Natriumbicarbonat |

ca. 10 bis 15 min gemischt.

Die resultierenden 199,875 kg Tablettiermischung werden auf einer Tablettenpresse unter externer Schmierung mit weniger als 0,125 kg Magnesiumstearat zu 50.000 Rundtabletten mit 25 mm Durchmesser und einem Tablettengewicht von 4 g verpreßt.

### Beispiel 6.2

### Vitamin C 500 mg Brausetablette

| | |
|---|---|
| 25,000 kg | Ascorbinsäure |
| 1,500 kg | Natriumcyclamat |
| 0,500 kg | Natriumsaccharinat |
| 0,075 kg | Riboflavin |
| 0,050 kg | Beta-Carotin |
| 6,000 kg | Sorbitol |
| 1,500 kg | Fruchtaroma |

werden im Intensivmischer 3 min gemischt.

0,250 kg Propylenglycol werden zugegeben und nochmals kurzzeitig gemischt.

Das Ascorbinsäure-Farbstoff-Süßstoff-Aromakonzentrat wird im klimatisierten Raum (relative Luftfeuchtigkeit < 25 %) in einem geeigneten Mischer mit

| | |
|---|---|
| 29,000 kg | Citronensäure |
| 36,000 kg | Natriumbicarbonat |

ca. 10 bis 15 min gemischt.

Die resultierenden 99,875 kg Tablettiermischung werden auf einer Tablettenpresse unter externer Schmierung mit weniger als 0,125 kg Silikonöl zu 50.000 Rundtabletten mit 20 mm Durchmesser und einem Tablettengewicht von 2 g verpreßt.

### Beispiel 6.3

### Vitamin C 1000 mg Brausetablette

| | |
|---|---|
| 50,000 kg | Ascorbinsäure |
| 2,500 kg | Natriumcyclamat |
| 0,500 kg | Natriumsaccharinat |
| 0,750 kg | Kollidon 30 |
| 4,000 kg | Maltodextrin |
| 8,500 kg | Sorbitol |
| 2,500 kg | Fruchtaroma |

werden im Intensivmischer 3 min gemischt.

0,500 kg Propylenglycol werden zugegeben und nochmals kurzzeitig gemischt.

Das Ascorbinsäure-Farbstoff-Süßstoff-Aromakonzentrat wird im klimatisierten Raum (relative Luftfeuchtigkeit < 25 %) in einem geeigneten Mischer mit

| | |
|---|---|
| 0,500 kg | Citronensäure |
| 32,400 kg | Mononatriumcitrat |
| 29,750 kg | Natriumbicarbonat |

ca. 10 bis 15 min gemischt.

Die resultierenden 131,900 kg Tablettiermischung werden auf einer Tablettenpresse unter externer Schmierung mit weniger als 0,100 kg Silikonöl zu 50.000 Rundtabletten mit 22 mm Durchmesser und einem Tablettengewicht von 2,64 g verpreßt.

### Beispiel 7.1

### Calcium-Gluconat Brausetablette

| | |
|---|---|
| 50,000 kg | Calciumgluconatmonohydrat |
| 2,000 kg | Natriumcyclamat |
| 0,650 kg | Natriumsaccharinat |
| 0,005 kg | Riboflavin |
| 4,000 kg | Karion Instant |
| 1,750 kg | Fruchtaroma |

werden im Intensivmischer 3 min gemischt.

0,750 kg Propylenglycol werden zugegeben und nochmals kurzzeitig gemischt.

Das Calcium-Farbstoff-Süßstoff-Aromakonzentrat wird im klimatisierten Raum (relative Luftfeuchtigkeit < 25 %) in einem geeigneten Mischer mit

| | |
|---|---|
| 97,000 kg | Citronensäure |
| 54,000 kg | Natriumbicarbonat |

ca. 10 bis 15 min gemischt.

Die resultierenden 210,200 kg Tablettiermischung werden auf einer Tablettenpresse unter externer Schmierung mit weniger als 0,050 kg Magnesiumstearat zu 50.000 Rundtabletten mit 25 mm Durchmesser und einem Tablettengewicht von 4,2 g verpreßt.

Die Tablettenhärte beträgt 40 bis 50 N.

### Beispiel 7.2

### Calcium 500 mg Brausetablette

| | |
|---|---|
| 150,000 kg | Calciumcarbonat |
| 4,800 kg | Natriumcyclamat |
| 1,440 kg | Natriumsaccharinat |
| 0,012 kg | Riboflavin |
| 3,600 kg | Karion Instant |
| 4,200 kg | Fruchtaroma |

werden im Intensivmischer 3 min gemischt.

3,600 kg Propylenglycol werden zugegeben und nochmals kurzzeitig gemischt.

Das Calcium-Farbstoff-Süßstoff-Aromakonzentrat wird im klimatisierten Raum (relative Luftfeuchtigkeit < 25 %) in einem geeigneten Mischer mit

| | |
|---|---|
| 433,788 kg | Citronensäure |
| 178,560 kg | Natriumbicarbonat |

ca. 10 bis 15 min gemischt.

Die resultierenden 779,800 kg Tablettiermischung werden auf einer Tablettenpresse unter externer Schmierung mit weniger als 0,200 kg Magnesiumstearat zu 120.000 Rundtabletten mit 25 mm Durchmesser und einem Tablettengewicht von 6,5 g verpreßt.

Die Tablettenhärte beträgt 40 bis 50 N.

### Beispiel 7.3

### Calcium 500 mg Brausetablette (ohne Natrium)

| | |
|---|---|
| 62,500 kg | Calciumcarbonat |
| 1,000 kg | Natriumcyclamat |
| 0,350 kg | Natriumsaccharinat |
| 2,000 kg | Maltodextrin |
| 1,500 kg | Fruchtaroma |

werden im Intensivmischer 3 min gemischt.

2,500 kg Propylenglycol werden zugegeben und nochmals kurzzeitig gemischt.

Das Calcium-Farbstoff-Süßstoff-Aromakonzentrat wird im klimatisierten Raum (relative Luftfeuchtigkeit < 25 %) in einem geeigneten Mischer mit

| | |
|---|---|
| 100,000 kg | Citronensäure |

ca. 10 bis 15 min gemischt.

Die resultierenden 169,850 kg Tablettiermischung werden auf einer Tablettenpresse unter externer Schmierung mit weniger als 0,150 kg Silikonöl zu 50.000 Rundtabletten mit 22 mm Durchmesser und einem Tablettengewicht von 3,4 g verpreßt.

### Beispiel 7.4

### Calcium + Vitamin C Brausetablette

| | |
|---|---|
| 150,000 kg | Calciumcarbonat |
| 15,000 kg | Ascorbinsäure |
| 5,400 kg | Natriumcyclamat |
| 1,440 kg | Natriumsaccharinat |
| 0,180 kg | Riboflavin |
| 2,400 kg | Rote Beete Pulver |
| 4,620 kg | Fruchtaroma |

werden im Intensivmischer 3 min gemischt.

3,000 kg Propylenglycol werden zugegeben und nochmals kurzzeitig gemischt.

Das Calcium-Vitamin C-Farbstoff-Süßstoff-Aromakonzentrat wird im klimatisierten Raum (relative Luftfeuchtigkeit < 25 %) in einem geeigneten Mischer mit

| | |
|---|---|
| 426,360 kg | Citronensäure |
| 171,600 kg | Natriumbicarbonat |

ca. 10 bis 15 min gemischt.

Die resultierenden 780,000 kg Tablettiermischung werden auf einer Tablettenpresse unter externer Schmierung mit weniger als 0,200 kg Magnesiumstearat zu 120.000 Rundtabletten mit 25 mm Durchmesser und einem Tablettengewicht von 6,5 g verpreßt.

Die Tablettenhärte beträgt 40 bis 50 N.

### Beispiel 8.1

### Magnesium 150 mg Brausetablette

| | |
|---|---|
| 140,400 kg | Magnesiumhydroxidcarbonat |
| 9,600 kg | Natriumcyclamat |
| 3,000 kg | Natriumsaccharinat |
| 0,048 kg | Riboflavin |
| 12,000 kg | Maltodextrin |
| 12,480 kg | Fruchtaroma |

werden im Intensivmischer 3 min gemischt.

7,200 kg Propylenglycol werden zugegeben und nochmals kurzzeitig gemischt.

Das Magnesium-Farbstoff-Süßstoff-Aromakonzentrat wird im klimatisierten Raum (relative Luftfeuchtigkeit < 25 %) in einem geeigneten Mischer mit

| | |
|---|---|
| 844,392 kg | Citronensäure |
| 530,880 kg | Natriumbicarbonat |

ca. 10 bis 15 min gemischt.

Die resultierenden 1559,800 kg Tablettiermischung werden auf einer Tablettenpresse unter externer Schmierung mit weniger als 0,200 kg Magnesiumstearat zu 240.000 Rundtabletten mit 25 mm Durchmesser und einem Tablettengewicht von 6,5 g verpreßt.

Die Tablettenhärte beträgt 50 bis 70 N.

### Beispiel 8.2

### Magnesium + Vitamin C Brausetablette

| | |
|---|---|
| 81,960 kg | Magnesiumcarbonat |
| 15,000 kg | Ascorbinsäure |
| 4,200 kg | Natriumcyclamat |
| 1,200 kg | Natriumsaccharinat |
| 12,000 kg | Maltodextrin |
| 4,800 kg | Fruchtaroma |

werden im Intensivmischer 3 min gemischt.

3,600 kg Propylenglycol werden zugegeben und nochmals kurzzeitig gemischt.

Das Magnesium-Vitamin C-Farbstoff-Süßstoff-Aromakonzentrat wird im klimatisierten Raum (relative Luftfeuchtigkeit < 25 %) in einem geeigneten Mischer mit

| | |
|---|---|
| 2,160 kg | Rote Beete Pulver |
| 0,720 kg | Riboflavin |
| 427,550 kg | Citronensäure |
| 226,800 kg | Natriumbicarbonat |

ca. 10 bis 15 min gemischt.

Die resultierenden 779,800 kg Tablettiermischung werden auf einer Tablettenpresse unter externer Schmierung mit weniger als 0,200 kg Magnesiumstearat zu 120.000 Rundtabletten mit 25 mm Durchmesser und einem Tablettengewicht von 6,5 g verpreßt.

Die Tablettenhärte beträgt 50 bis 70 N.

### Beispiel 9

### Vitamin E Brausetablette

| | |
|---|---|
| 7,500 kg | Vitamin E Acetat |
| 1,250 kg | Natriumsaccharinat |
| 28,000 kg | Maltodextrin |
| 1,250 kg | Rote Beete Pulver |
| 0,250 kg | Riboflavin |
| 2,000 kg | Karion Instant |
| 1,250 kg | Fruchtaroma |

werden im Intensivmischer 3 min gemischt.

0,125 kg Propylenglycol werden zugegeben und nochmals kurzzeitig gemischt.

Das Vitamin E-Farbstoff-Süßstoff-Aromakonzentrat wird im klimatisierten Raum (relative Luftfeuchtigkeit < 25 %) in einem geeigneten Mischer mit

| | |
|---|---|
| 97,250 kg | Citronensäure |
| 66,000 kg | Natriumbicarbonat |

ca. 10 bis 15 min gemischt.

Die resultierenden 204,875 kg Tablettiermischung werden auf einer Tablettenpresse unter externer Schmierung mit weniger als 0,125 kg Silikonöl zu 50.000 Rundtabletten mit 25 mm Durchmesser und einem Tablettengewicht von 4,1 g verpreßt.

### Beispiel 10.1

### Multivitamin Brausetablette

| | |
|---|---|
| 72,000 kg | Multivitaminmischung |
| 6,300 kg | Natriumcyclamat |
| 1,980 kg | Natriumsaccharinat |
| 40,500 kg | Karion Instant |
| 24,300 kg | Maltodextrin |
| 4,500 kg | Fruchtaroma |

werden im Intensivmischer 3 min gemischt.

1,260 kg Propylenglycol werden zugegeben und nochmals kurzzeitig gemischt.

Das Multivitamin-Farbstoff-Süßstoff-Aromakonzentrat wird im klimatisierten Raum (relative Luftfeuchtigkeit < 25 %) in einem geeigneten Mischer mit

| | |
|---|---|
| 2,700 kg | Rote Beete Pulver |
| 404,460 kg | Citronensäure |
| 252,000 kg | Natriumbicarbonat |

ca. 10 bis 15 min gemischt.

Die resultierenden 809,500 kg Tablettiermischung werden auf einer Tablettenpresse unter externer Schmierung mit weniger als 0,500 kg Magnesiumstearat zu 180.000 Rundtabletten mit 25 mm Durchmesser und einem Tablettengewicht von 4,5 g verpreßt.

Die Tablettenhärte beträgt 70 bis 80 N.

### Beispiel 10.2

### Multivitamin + Mineral Brausetablette

| | |
|---|---|
| 72,000 kg | Multivitaminmischung |
| 45,000 kg | Calciumcarbonat |
| 79,200 kg | Kaliumhydrogenphosphat |
| 21,870 kg | Magnesiumcarbonat |
| 6,300 kg | Natriumsaccharinat |
| 18,900 kg | Maltodextrin |
| 9,000 kg | Fruchtaroma |

werden im Intensivmischer 3 min gemischt.

4,500 kg Propylenglycol werden zugegeben und nochmals kurzzeitig gemischt.

Das Multivitamin-Mineral-Farbstoff-Süßstoff-Aromakonzentrat wird im klimatisierten Raum (relative Luftfeuchtigkeit < 25 %) in einem geeigneten Mischer mit

| | |
|---|---|
| 3,600 kg | Rote Beete Pulver |
| 400,500 kg | Citronensäure |
| 108,000 kg | Natriumbicarbonat |
| 24,300 kg | Natriumcarbonat |
| 16,830 kg | Kaliumbicarbonat |

ca. 10 bis 15 min gemischt.

Die resultierenden 809,500 kg Tablettiermischung werden auf einer Tablettenpresse unter externer Schmierung mit weniger als 0,500 kg Magnesiumstearat zu 180.000 Rundtabletten mit 25 mm Durchmesser und einem Tablettengewicht von 4,5 g verpreßt.

Die Tablettenhärte beträgt 80 bis 90 N.

### Beispiel 11

### Carotin + Vitamin C+E Brausetablette

| | |
|---|---|
| 14,000 kg | Protectormischung |
| 3,750 kg | Ascorbinsäure |
| 0,500 kg | Calciumpantothenat |
| 31,250 kg | Calciumcarbonat |
| 8,750 kg | Magnesiumcarbonat |
| 0,0001kg | Biotin |
| 1,500 kg | Natriumcyclamat |
| 0,750 kg | Natriumsaccharinat |
| 2,500 kg | Fruchtaroma |

werden im Intensivmischer 3 min gemischt.

1,000 kg Propylenglycol werden zugegeben und nochmals kurzzeitig gemischt.

Das Carotin-Vitamin E+C-Farbstoff-Süßstoff-Aromakonzentrat wird im klimatisierten Raum (relative Luftfeuchtigkeit < 25 %) in einem geeigneten Mischer mit

| | |
|---|---|
| 126,000 kg | Citronensäure |
| 30,000 kg | Natriumbicarbonat |

ca. 10 bis 15 min gemischt.

Die resultierenden 219,875 kg Tablettiermischung werden auf einer Tablettenpresse unter externer Schmierung mit weniger als 0,125 kg Silikonöl zu 50.000 Rundtabletten mit 25 mm Durchmesser und einem Tablettengewicht von 4,4 g verpreßt. Dabei enthalten 280 mg Protectormischung pro Brausetablette 6 mg Beta-Carotin, 12 mg Vitamin E und 75 mg Vitamin C.

### Beispiel 12

### Eisen + Vitamin C Brausetablette

| | |
|---|---|
| 38,850 kg | Eisen-II-gluconatdihydrat |
| 31,250 kg | Ascorbinsäure |
| 0,0015kg | Vitamin B12 |
| 0,050 kg | Folsäure |
| 7,500 kg | Natriumcyclamat |
| 4,500 kg | Natriumsaccharinat |
| 125,000 kg | Maltodextrin |
| 15,000 kg | Karion Instant |
| 5,000 kg | Fruchtaroma |

werden im Intensivmischer 3 min gemischt.

1,250 kg Propylenglycol werden zugegeben und nochmals kurzzeitig gemischt.

Das Eisen-Vitamin C-Farbstoff-Süßstoff-Aromakonzentrat wird im klimatisierten Raum (relative Luftfeuchtigkeit < 25 %) in einem geeigneten Mischer mit

| | |
|---|---|
| 0,250 kg | Riboflavin |
| 11,250 kg | Rote Beete Pulver |
| 810,100 kg | Citronensäure |
| 575,000 kg | Natriumbicarbonat |

ca. 10 bis 15 min gemischt.

Die resultierenden 1624,502 kg Tablettiermischung werden auf einer Tablettenpresse unter externer Schmierung mit weniger als 0,500 kg Magnesiumstearat zu 250.000 Rundtabletten mit 25 mm Durchmesser und einem Tablettengewicht von 6,5 g verpreßt.

Die Tablettenhärte beträgt 45 bis 60 N. In der nachstehenden Übersicht 1 sind die Auflösungszeiten der in den Beispielen 1 bis 12 hergestellten Brausetabletten aufgeführt. Dort findet sich außerdem die Angabe des Anteils des Wirkstoffs bzw. der Wirkstoffkombination und der Trägerstoffe sowie des Anteils des Bindemittels bezogen auf das Gesamtgewicht der Brausetabletten.

Unter der Auflösungszeit ist der Zeitraum zu verstehen, in welchem bei einer in Wasser eingebrachten Brausetablette die Gasentwicklung abgeschlossen und die Tablette gelöst ist, ohne daß eine Anhäufung von Teilchen bzw. Partikeln zurückbleibt. Die Erfassung der Auflösungszeit in Sekunden erfolgt durch Einbringen von jeweils einer Tablette in 150 ml Wasser von 20°C. Dabei erfolgt die rechnerische Erfassung der Auflösungszeit über das Prinzip der Mittelwertbildung.

Aus Übersicht 1 ist festzustellen, daß sich nach dem erfindungsgemäßen Verfahren hergestellte Brausetabletten mit einer wesentlich geringeren Zerfallzeit im Vergleich zu der nach Ph. Eu. 2, (Europäische Pharmaziebuch, 2. Ausgabe) angegebenen Zerfallzeit von 300 s auszeichnen.

Weiterhin ist ersichtlich, daß der Anteil der Brausezusätze, der mit den neuen Bindemitteln zuverlässig in mechanisch stabile Brausetabletten einbringbar ist, sehr groß ist. Dies ist neben den Eigenschaften des Bindemittels selbst ein Grund für das rasche Auflösen der Brausetabletten. Wenn der Anteil der Brausezusätze als Bereich angegeben ist, bedeutet dies, daß der Wirkstoff oder Teile der Wirkstoffkombination auch eine Funktion als Brausezusatz haben.

Umgekehrt ist der Anteil des Wirkstoffs bzw. der Wirkstoffkombination, der Trägerstoffe und des Bindemittels, d.h. der Anteil, der im Intensivmischer gemischt werden muß, bezogen auf das Gesamtgewicht der Brausetabletten im Vergleich zu den aus den Literatur bekannten Verhältnissen relativ gering. Dies bedeutet, daß eine gute Dosierbarkeit der Wirkstoffe bzw. der Wirkstoffkombinationen gegeben ist.

Der Anteil des Bindemittels an den neuen Brausetabletten ist extrem gering.

### Übersicht 1

### Brausetabletten

| Beispiel | Auflösezeit [ s ] | Anteil der Brausezusätze [ % ] | Anteil des Bindemittels [ % ] |
|---|---|---|---|
| 1.1 | <60 | 78,89 | 0,0185 |
| 1.2 | <120 | 80,97 | 0,0139 |
| 2.1 | 150 | 69,33 | 0,1000 |
| 2.2 | <120 | 62,73 | 0,0998 |
| 3.1 | <60 | 94,47 | 0,0052 |
| 3.2 | <120 | 83,56 | 0,0042 |
| 4.1 | <70 | 78,21 | 0,1339 |
| 4.2 | <70 | 78,43 | 0,1429 |
| 5 | <100 | 74,21 | 0,3145 |
| 6.1 | 120 | 82,07 | 0,0625 |
| 6.2 | 180 | 65,00 | 0,2500 |
| 6.3 | 150 | 47,46-85,34 | 0,3788 |
| 7.1 | 100 | 71,90 | 0,3567 |
| 7.2 | 100 | 78,51 | 0,4615 |
| 7.3 | 180 | 58,82-95,59 | 1,4706 |
| 7.4 | 60 | 76,66 | 0,3846 |
| 8.1 | 100 | 88,16 | 0.4615 |
| 8.2 | 120 | 83,89 | 0,4615 |
| 9 | 90 | 79,63 | 0,0610 |
| 10.1 | 60 | 81,04 | 0,1556 |
| 10.2 | 90 | 67,86 | 0.5556 |
| 11 | 100 | 70,91 | 0,4545 |
| 12 | 45 | 85,23 | 0,0769 |

## Patentansprüche

1. Verfahren zur Herstellung von Brausetabletten bestehend aus
- mindestens einem Wirkstoff oder einer Wirkstoffkombination,
- mindestens einem Bindemittel,
- gegebenenfalls Trägerstoffen, wie Süßstoffen, Aromen, Farbstoffen, Duftstoffen, Weichmachern, Bleichmittel, und
- Brausezusätzen,
wobei der Wirkstoff oder die Wirkstoffkombination und gegebenenfalls die Trägerstoffe mit dem Bindemittel vermischt werden, wobei zu dieser Mischung in einem klimatisierten Raum die Brausezusätze zugegeben werden und wobei die mit den Brausezusätzen versetzte Mischung tablettiert wird,
**dadurch gekennzeichnet,** daß als Bindemittel Propylenglykol oder Glycerin ohne Zusatz von Lösungsmitteln mit einem Bindemittelanteil von 0,004 bis 2,5 % des Gesamtgewichtes der Brausetablette verwendet wird.

2. Verfahren nach Anspruch 1, **dadurch gekennzeichnet,** daß der Wirkstoff oder die Wirkstoffkombination und gegebenenfalls die Trägerstoffe mit dem Bindemittel intensiv vermischt werden, bevor die Brausezusätze zugegeben werden.

3. Verfahren nach Anspruch 1 oder 2, **dadurch gekennzeichnet,** daß die mit den Brausezusätzen versetzte Mischung direkt tablettiert wird.

4. Verfahren nach einem der Ansprüche 1 bis 3, **dadurch gekennzeichnet,** daß der Bindemittelanteil 0,004 bis 1,5 % des Gesamtgewichtes der Brausetabletten beträgt.

5. Verfahren nach Anspruch 4, **dadurch gekennzeichnet,** daß der Bindemittelanteil 0,01 bis 1,0 % des Gesamtgewichts der Brausetabletten beträgt.

6. Verfahren nach einem der Ansprüche 1 bis 3, **dadurch gekennzeichnet,** daß der Bindemittelanteil 0,05 bis 2,5 % des Gesamtgewichts der Brausetabletten beträgt.

7. Verfahren nach einem der Ansprüche 4 bis 6, **dadurch gekennzeichnet,** daß der Anteil der Brausezusätze 58 bis 93 % des Gesamtgewichts der Brausetabletten beträgt.

8. Verfahren nach Anspruch 7, **dadurch gekennzeichnet,** daß der Anteil der Brausezusätze 70 bis 90 % des Gesamtgewichts der Brausetabletten beträgt.

## Claims

1. A method of producing effervescent tablets which consist of
- at least one active substance or a combination of active substances,
- at least one binder,
- possibly carriers as sweeteners, flavours, colourings, scents, softeners, bleaches, and
- sherbets,
wherein the active substance or the combination of active substances and possibly the carriers are mixed with the binder, wherein the sherbets are added to this mixture in an airconditioned room and wherein the mixture including the sherbets is formed into tablets,
**characterized in that** propylglycol or glycerin is used without addition of solvents as binder with an amount of binder of 0.004 to 2.5 % per weight of the whole effervescent tablet.

2. A method according to claim 1, **characterized in that** the active substance or the combination of active substances and possibly the carriers are intensely mixed before adding the sherbets.

3. A method according to claim 1 or 2, **characterized in that** the mixture including the sherbets is directly formed into tablets.

4. A method according to any one of claims 1 to 3, **characterized in that** the amount of binder is 0.004 to 1.5 % per weight of the whole effervescent tablet.

5. A method according to claim 4, **characterized in that** the amount of binder is 0.01 to 1.0 % per weight of the whole effervescent tablet.

6. A method according to any one of claims 1 to 3, **characterized in that** the amount of binder is 0.05 to 2.5 % per weight of the whole effervescent tablet.

7. A method according to any one of the claims 4 to 6, **characterized in that** the amount of sherbets is 58 to 93 % per weight of the whole effervescent tablet.

8. A method according to claim 7, **characterized in that** the amount of sherbets is 70 to 90 % per weight of the whole effervescent tablet.

## Revendications

1. Procédé pour la fabrication de comprimés effervescents constitués
- d'au moins une substance active ou une association de substances actives,
- d'au moins un liant,
- éventuellement de véhicules, tels qu'édulcorants, arômes, colorants, parfums, plastifiants, agents de blanchiment et
- d'additifs effervescents,
dans lequel on mélange avec le liant la substance active ou l'association de substances actives et éventuellement les véhicules, en ajoutant: à ce mélange les additifs effervescents, dans une pièce climatisée, et on transforme en comprimés le mélange additionné des additifs effervescents, **caractérisé en ce qu**'on utilise comme liant du propylèneglycol ou du glycérol sans addition de solvants, avec une proportion de liant représentant de 0,004 à 2,5 % du poids total du comprimé effervescent.

2. Procédé selon la revendication 1, **caractérisé en ce que** la substance active ou l'association de substances actives et éventuellement les véhicules sont intimement mélangés avec le liant, avant l'addition des additifs effervescents.

3. Procédé selon la revendication 1 ou 2, **caractérisé en ce que** le mélange additionné des additifs effervescents est transformé directement en comprimés.

4. Procédé selon l'une quelconque des revendications 1 à 3, **caractérisé en ce que** la proportion du liant représente de 0,004 à 1,5 % du poids total des comprimés effervescents.

5. Procédé selon la revendication 4, **caractérisé en ce que** la proportion du liant représente de 0,01 à 1,0 % du poids total des comprimés effervescents.

6. Procédé selon l'une quelconque des revendications 1 à 3, **caractérisé en ce que** la proportion du liant représente de 0,05 à 2,5 % du poids total des comprimés effervescents.

7. Procédé selon l'une quelconque des revendications 4 à 6, **caractérisé en ce que** la proportion des additifs effervescents représente de 58 à 93 % du poids total des comprimés effervescents.

8. Procédé selon la revendication 7, **caractérisé en ce que** la proportion des additifs effervescents représente de 70 à 90 % du poids total des comprimés effervescents.
